# FASCICULE DE BREVET EUROPEEN

(11) **EP 3 041 486 B1**
(45) Date de publication et mention de la délivrance du brevet: **24.04.2019**
(21) Numéro de dépôt: 14739874.7
(22) Date de dépôt: 11.06.2014
(51) Int. Cl.: A61K 35/64, A23L 21/20, A61K 36/28, A61K 36/76, A61P 35/00, A61P 43/00

(54) **UTILISATION DE PROPOLIS POUR LUTTER CONTRE LES EFFETS SECONDAIRES DES CHIMIOTHERAPIES**
VERWENDUNG VON PROPOLIS GEGEN DIE NEBENWIRKUNGEN DER CHEMOTHERAPIE
USE OF PROPOLIS AGAINST THE SIDE EFFECTS OF CHEMOTHERAPY

(30) Priorité: 11.06.2013 FR 1355356
(43) Date de publication de la demande: 13.07.2016
(73) Titulaire: Pollenergie, 47450 Saint-Hilaire-de-Lusignan (FR)
(72) Inventeur: CARDINAULT, Nicolas, F-47000 Agen (FR)
(74) Mandataire: Aquinov
(86) Numéro de dépôt international: PCT/FR2014/051407
(87) Numéro de publication internationale: WO 2014/199076

(56) Documents cités:
- WO-A1-2013/012477
- CN-A- 1 857 323
- CN-B- 102 048 959
- JP-A- 2013 023 498
- ORSOLIC N ET AL: "Antitumor, hematostimulative and radioprotective action of water-soluble derivative of propolis (WSDP)", BIOMEDICINE AND PHARMACOTHERAPY, ELSEVIER, FR, vol. 59, no. 10, 1 décembre 2005 (2005-12-01), pages 561-570, XP027640259, ISSN: 0753-3322 [extrait le 2005-12-01]
- SUZUKI I ET AL: "Antitumor and anticytopenic effects of aqueous extracts of propolis in combination with chemotherapeutic agents", CANCER BIOTHERAPY & RADIOPHARMACEUTICALS, MARY ANN LIEBERT, US, vol. 17, no. 5, 1 janvier 2002 (2002-01-01), pages 553-562, XP002505357, ISSN: 1084-9785, DOI: 10.1089/108497802760804781
- LEANDRO L MOREIRA ET AL: "Propolis influence on erythrocyte membrane disorder (hereditary spherocytosis): A first approach", FOOD AND CHEMICAL TOXICOLOGY, PERGAMON, GB, vol. 49, no. 2, 29 novembre 2010 (2010-11-29), pages 520-526, XP028128214, ISSN: 0278-6915, DOI: 10.1016/J.FCT.2010.11.042 [extrait le 2010-12-03]
- M LAHOUEL ET AL: "Effet protecteur des flavonoïdes contre la toxicité de la vinblastine, du cyclophosphamide et du paracétamol par inhibition de la peroxydation lipidique et augmentation du glutathion hépatique", PATHOLOGIE BIOLOGIE, vol. 52, no. 6, 1 juillet 2004 (2004-07-01), pages 314-322, XP055082656, ISSN: 0369-8114, DOI: 10.1016/j.patbio.2004.01.001

## Description

La présente invention concerne la prévention et la lutte contre les effets secondaires des chimiothérapies.

La chimiothérapie est l'un des principaux traitements du cancer. Il s'agit d'un traitement agressif qui permet de s'attaquer aux cellules cancéreuses disséminées dans l'organisme. Toutefois, l'agression des cellules cancéreuses n'est pas suffisamment ciblée et entraîne très souvent des effets secondaires désagréables et néfastes pour le patient, tels qu'une diminution des globules rouges, des globules blancs et des plaquettes, des nausées et vomissements, un état de fatigue important, la chute des cheveux et des ongles, etc.

Il est donc nécessaire de trouver une solution efficace pouvant prévenir et limiter le plus possible les effets secondaires qui découlent des traitements par chimiothérapie. Actuellement, il n'existe pas de solution satisfaisante. Dans le cas d'une diminution trop importante des globules rouges, les oncologues doivent retarder le protocole de chimiothérapie initialement prévu pour laisser à l'organisme le temps de re-synthétiser ses globules rouges ou doivent accélérer cette synthèse en injectant un facteur de croissance, l'EPO qui présente l'inconvénient de favoriser la croissance des cellules cancéreuses également.

L'objectif de l'invention est de proposer une solution qui d'une part prépare l'organisme et notamment les cellules saines à être agressées par l'agent de chimiothérapie et à mieux lui résister, pour limiter au maximum les effets secondaires qui en découlent, et d'autre part à ne pas interférer avec l'efficacité de l'agent de chimiothérapie.

Pour y répondre, l'invention propose d'utiliser une composition comprenant au moins un extrait de propolis de peuplier contenant des polyphénols.

En particulier, l'invention vise une composition comprenant au moins un extrait de propolis de peuplier contenant des polyphénols, pour son application comme produit de santé, en particulier comme complément nutritionnel oral humain ou comme médicament pour prévenir et/ou limiter les effets secondaires des chimiothérapies. Préférentiellement, la composition est un Aliment Diététique Destiné à des Fins Médicales Spéciales (ADDFMS).

La propolis est un produit fabriqué par les abeilles à partir de substances résineuses, gommeuses et balsamiques, récoltées sur les bourgeons de certains arbres et arbustes auxquelles elles y incorporent des sécrétions salivaires.

De façon surprenante, selon l'invention, la propolis de peuplier présente une bonne efficacité pour prévenir et/ou limiter les effets secondaires des chimiothérapies.

Avantageusement, l'administration à un patient sous chimiothérapie, d'une composition comprenant au moins un extrait de propolis de peuplier contenant des polyphénols permet de préserver leur qualité de vie.

L'invention est maintenant décrite en détail.

L'invention vise donc une composition comprenant au moins un extrait de propolis de peuplier contenant des polyphénols, pour son utilisation comme produit de santé, en particulier comme complément nutritionnel oral humain ou comme médicament, pour prévenir et/ou limiter les effets secondaires des chimiothérapies.

Par extrait de propolis on entend toute propolis de peuplier récoltée transformée par un procédé d'extraction permettant d'enlever les impuretés présentes dans l'extrait brut et/ou de concentrer la propolis en un ou plusieurs de ses constituants.

L'extrait de propolis peut se présenter sous toute forme. Préférentiellement, il se présente sous forme de poudre.

L'extrait de propolis de peuplier utile selon l'invention est un extrait de propolis comprenant au moins 20% de polyphénols en poids de matière sèche de l'extrait.

L'extrait de propolis de peuplier comprend préférentiellement au moins 30% de polyphénols en poids de matière sèche de l'extrait.

L'extrait présent dans la composition peut être obtenu par un procédé comprenant les étapes suivantes :
- extraction de propolis de peuplier,
- concentration.

L'extrait peut ensuite être reconcentré et enfin transformé.

Les différentes étapes du procédé doivent être réalisées, sans détruire les principes actifs et sans utiliser de solvants.

Selon un mode de réalisation particulièrement adapté, l'extrait utilisé selon l'invention peut être obtenu par un procédé comprenant les étapes suivantes :
- macération de propolis de peuplier brute dans une solution alcoolique, et
- concentration par évaporation.

L'extrait peut ensuite être transformé en poudre.

L'extrait de propolis de peuplier obtenu est incorporé dans une composition.

La composition comprenant l'extrait de propolis de peuplier est utilisée comme complément nutritionnel oral humain ou comme médicament.

Préférentiellement, la composition selon l'invention est un complément nutritionnel en particulier un Aliment Diététique Destiné à des Fins Médicales Spéciales (ADDFMS).

La composition selon l'invention, en plus de l'extrait de propolis de peuplier, peut contenir des protéines, des glucides, des lipides, des vitamines et/ou des minéraux, ajoutés en suppléments. Préférentiellement ces molécules sont ajoutées selon la règlementation en vigueur sur les ADDFMS.

La composition peut également contenir des excipients connus de l'homme de l'art, tels que du kaolin ou du fibregum® ou d'autres agents texturants, d'enrobage et de gastro-résistance connus dans le domaine pharmaceutique.

Selon un mode de réalisation adapté, la composition se présente sous forme d'une poudre micro-encapsulée.

La composition selon l'invention, lorsqu'il s'agit d'un complément nutritionnel oral est administrée en plus et/ou en substitut des repas.

De façon préférée, la dose journalière de composition comprend entre 600 et 1200 mg d'extrait de propolis de peuplier en poids de matière sèche. Préférentiellement, la dose journalière est répartie en 2 ou 3 prises.

La composition selon l'invention comprenant au moins un extrait de propolis de peuplier contenant des polyphénols est utilisée pour son application pour prévenir et/ou limiter les effets secondaires des chimiothérapies.

En particulier, la composition selon l'invention peut être utilisée pour préserver et/ou limiter la diminution des globules blancs, des globules rouges et des plaquettes sanguines consécutives aux injections d'agents de chimiothérapies.

En effet, l'injection d'agents de chimiothérapies provoque une diminution des globules blancs, des globules rouges et des plaquettes sanguines, et l'utilisation d'un extrait de propolis contenant des polyphénols permet de limiter cette diminution.

En outre, la composition selon l'invention peut être utilisée pour préserver et/ou limiter les dégâts radicalaires causés par les agents de chimiothérapies sur des organes, en particulier sur le foie, les reins et/ou le coeur.

Avantageusement, grâce à son action notamment sur la limitation de la diminution des globules blancs, des globules rouges et des plaquettes sanguines, et sur la limitation des dégâts radicalaires, la composition selon l'invention permet de prévenir et/ou lutter contre les nausées, chutes de cheveux, chutes d'ongles, états de fatigue et déprimes des personnes sous traitement chimiothérapeutique.

L'invention est à présent illustrée par des exemples et des résultats d'essais.

### Exemples d'extraits de propolis

La propolis de peuplier utilisée peut être obtenue par la mise en oeuvre de la technique des grilles qui permet d'obtenir une propolis avec des caractéristiques particulières adaptées à une utilisation médicale en comparaison d'une propolis obtenue par la technique de grattage. La méthode des grilles permet d'obtenir une propolis avec un taux de polyphénols plus important et un pourcentage de cire diminué.

Préférentiellement, le pourcentage de cire dans la propolis récoltée est inférieure à 21%, encore plus préférentiellement inférieur à 17%.

Une fois la propolis récoltée, elle est traitée par la mise en oeuvre d'un procédé d'extraction comprenant les étapes suivantes :
- la propolis est mélangée dans un extracteur avec de l'alcool suivant un ratio de 1/2,5 à 1/5 (w/v) pendant un laps de temps déterminé,
- le mélange subit ensuite une filtration afin de ne garder en solution liquide que les principes actifs de la propolis : les polyphénols,
- une dernière clarification par décantation gravitaire peut éventuellement être réalisée si nécessaire.

Ce procédé permet l'élaboration d'un extrait liquide concentré en principes actifs.

Cet extrait liquide peut ensuite éventuellement être encore concentré en principes actifs par desalcoolisation. On obtient alors un extrait mou très concentré en principes actifs de propolis. Cet extrait mou peut être transformé en poudre.

### Exemple 1

Un exemple d'extrait de propolis obtenu par la mise en oeuvre de ce procédé est un extrait de propolis de peuplier sous forme de poudre, présentant au moins 30% de polyphénols totaux en poids de matière sèche.

Parmi les polyphénols, l'extrait comprend notamment :
- au moins 8% (±0,8%) de pinocembrine,
- au moins 5% (±0,5%) de chrysine,
- au moins 4% (±0,4%) de galangine, et
- au moins 1,8% (±0,18%) de CAPE.
les pourcentages étant donnés en poids de matière sèche par rapport aux polyphénols totaux présents dans l'extrait.

### Exemple de compositions

Un exemple de composition utile selon l'invention comprend :
- 50 à 80% d'extrait de propolis de peuplier comprenant au moins 20% de polyphénols, et
- 10 à 15% de fibregum, et/ou,
- 10 à 15% de silice, et/ou,
- 10 à 15% de kaolin, et/ou,
- 10 à 15% d'au moins un autre excipient.

### Evaluation de l'effet de la propolis sur les effets secondaires des chimiothérapies

L'objectif de l'essai est d'évaluer l'effet de la propolis de peuplier sur la cytolyse de cellules souches CD34+ progenetrices issues de moelle osseuse humaine suite à l'application de traitement de chimiothérapie (épirubicine et taxotère).

Le milieu de culture utilisé est le suivant : IMDM, 15 SVF, SCF (50ng/ml), TPO (50ng/ml), FK (50ng/ml).

Le protocole de l'essai est décrit en suivant.
A J-3 : décongélation et ensemencement à 50 000 cellules/ml en flasque (75cm³)
A J0 : marquage et ensemencement à 6000 cellules/puits en plaque de 96 puits Entre J0 et J4 :
   o traitement des cellules avec un extrait de propolis (exemple 1) entre J0 et J3 aux doses suivantes : 0, 0,5, 1, 4, 10 et 20 µg/mL
   o traitement des cellules avec des agents de chimiothérapie (épirubicine à 4,74.10⁶ M et taxotère 2,80.10⁶ M) entre J1 et J3
A J5 : marquage avec 5nM de sytox green, et mesure de la cytolyse.

Les résultats obtenus présentés dans le tableau ci-dessous, donnent le pourcentage de cellules souches progénétrices CD34+ cytolysé à J5.

| **Propolis µg/ml** | Taxotère | Epirubicine |
|---|---|---|
| **0 (contrôle)** | 65,47 | 53,93 |
| **0,5** | 62,50 | 44,77 |
| **1** | 55,40 | 32,30 |
| **5** | 45,03 | 28,07 |
| **10** | 46,33 | 29,83 |
| **20** | 43,40 | 42,57 |

Ces résultats montrent qu'un extrait de propolis de peuplier concentré en polyphénols présente un effet cytoprotecteur vis-à-vis des effets cytotoxiques d'agents de chimiothérapie comme l'épirubicine et le taxotère sur des lignées progénétrices hématopoïétiques.

| **Propolis µg/ml** | Taxotère | Epirubicine |
|---|---|---|
| **0(contrôle)** | 65,47 | 53,93 |
| **0,5** | 62,50 | 44,77 |
| **1** | 55,40 | 32,30 |
| **5** | 45,03 | 28,07 |
| **10** | 46,33 | 29,83 |
| **20** | 43,40 | 42,57 |

Ces résultats montrent qu'un extrait de propolis concentré en polyphénols présente un effet cytoprotecteur vis-à-vis des effets cytotoxiques d'agents de chimiothérapie comme l'épirubicine et le taxotère sur des lignées progénétrices hématopoïétiques.

## Revendications

1. Composition comprenant au moins un extrait de propolis de peuplier comprenant au moins 20% de polyphénols en poids de matière sèche, pour son utilisation comme produit de santé pour prévenir et/ou limiter les effets secondaires des chimiothérapies.

2. Composition pour une utilisation selon la revendication 1, **caractérisée en ce que** le produit de santé est un complément nutritionnel oral humain ou comme médicament.

3. Composition pour une utilisation selon l'une des précédentes revendications, pour limiter et/ou préserver de la diminution des globules blancs, des globules rouges et des plaquettes sanguines consécutives aux injections d'agents de chimiothérapies.

4. Composition pour une utilisation selon l'une des précédentes revendications, pour préserver et/ou limiter les dégâts radicalaires causés par les agents de chimiothérapies sur des organes.

5. Composition pour une utilisation selon l'une des précédentes revendications, pour préserver et/ou limiter les dégâts radicalaires causés par les agents de chimiothérapies sur le foie, les reins et/ou le coeur.

6. Composition pour une utilisation selon l'une des précédentes revendications, pour prévenir et/ou lutter contre les nausées, chutes de cheveux, chutes d'ongles, états de fatigue et déprimes des personnes sous traitement chimiothérapeutique.

7. Composition pour une utilisation selon l'une des précédentes revendications, **caractérisée en ce que** l'extrait est un extrait de propolis de peuplier comprenant au moins 30% de polyphénols en poids de matière sèche.

8. Composition pour une utilisation selon l'une des précédentes revendications, **caractérisée en ce qu'**il s'agit d'un Aliment Diététique Destiné à des Fins Médicales Spéciales.

9. Composition pour une utilisation selon l'une des précédentes revendications, **caractérisée en ce qu'**elle comprend également des protéines, des glucides, des lipides, des vitamines et/ou des minéraux.

10. Composition pour une utilisation selon l'une des précédentes revendications, **caractérisée en ce qu'**il se présente sous forme de poudre.

11. Composition pour une utilisation selon l'une des précédentes revendications, **caractérisée en ce qu'**elle est administrée en plus et/ou en substitut des repas.

## Patentansprüche

1. Zusammensetzung mit wenigstens einem Extrakt aus Bienenharz von Pappeln mit wenigstens 20% Polyphenol im Trockengewicht, zu deren Verwendung als Gesundheitsprodukt zur Prävention und/oder Begrenzung von Nebenwirkungen der Chemotherapie.

2. Zusammensetzung zu deren Verwendung nach Anspruch 1, **dadurch gekennzeichnet, dass** das Gesundheitsprodukt ein orales Nahrungsergänzungsmittel für Menschen oder ein Medikament ist.

3. Zusammensetzung zu deren Verwendung nach einem der vorhergehenden Ansprüche, um die Verringerung der weißen Blutkörperchen, der roten Blutkörperchen und der Blutplättchen infolge von Injektionen von chemotherapeutischen Wirkstoffen zu begrenzen und/oder zu verhindern.

4. Zusammensetzung zu deren Verwendung nach einem der vorhergehenden Ansprüche, um durch Radikale an den Organen hervorgerufenen Schäden, die durch die chemotherapeutischen Wirkstoffe verursacht werden, zu verhindern und/oder zu begrenzen.

5. Zusammensetzung zu deren Verwendung nach einem der vorhergehenden Ansprüche, um die durch Radikale an der Leber, den Nieren und/oder dem Herz hervorgerufene Schäden, die durch die chemotherapeutischen Wirkstoffe verursacht werden, zu verhindern und/oder zu begrenzen.

6. Zusammensetzung zu deren Verwendung nach einem der vorhergehenden Ansprüche, zur Prävention und/oder Bekämpfung von Übelkeit, Haarausfall, Nagelausfall, Ermüdungszuständen und depressiven Stimmungen von Personen in chemotherapeutischer Behandlung.

7. Zusammensetzung zu deren Verwendung nach einem der vorhergehenden Ansprüche, **dadurch gekennzeichnet, dass** der Extrakt ein Extrakt aus Bienenharz von Pappeln mit wenigstens 30% Polyphenol im Trockengewicht ist.

8. Zusammensetzung zu deren Verwendung nach einem der vorhergehenden Ansprüche, **dadurch gekennzeichnet, dass** es sich um ein speziellen medizinischen Zwecken dienendes diätetisches Lebensmittel handelt.

9. Zusammensetzung zu deren Verwendung nach einem der vorhergehenden Ansprüche, **dadurch gekennzeichnet, dass** diese auch Proteine, Kohlenhydrate, Lipide, Vitamine und/oder Mineralstoffe enthält.

10. Zusammensetzung zu deren Verwendung nach einem der vorhergehenden Ansprüche, **dadurch gekennzeichnet, dass** diese in Gestalt eines Pulvers vorliegt.

11. Zusammensetzung zu deren Verwendung nach einem der vorhergehenden Ansprüche, **dadurch gekennzeichnet, dass** diese in Ergänzung zu und/oder als Ersatz von Mahlzeiten verabreicht wird.

## Claims

1. A composition comprising at least an extract of poplar propolis, comprising at least 20% polyphenols by weight of dry matter, for use as a health product for preventing and/or limiting the secondary effects of chemotherapy.

2. A composition for use according to claim 1, **characterised in that** the health product is a human oral nutritional supplement or a medication.

3. A composition for use according to either of the preceding claims, for limiting and/or protecting from reduction in white corpuscles, red corpuscles and blood platelets following injection of a chemotherapy agent.

4. A composition for use according to any of the preceding claims, for protecting from and/or limiting radical damage caused by a chemotherapy agents on organ.

5. A composition for use according to any of the preceding claims, for protecting from and/or limiting radical damage caused by a chemotherapy agent on the liver, the kidneys and/or the heart.

6. A composition for use according to any of the preceding claims, for preventing and/or combating nausea, hair loss, nail loss, and fatigue and depression states in persons under chemotherapy treatment.

7. A composition for use according to any of the preceding claims, **characterised in that** the extract is an extract of poplar propolis comprising at least 30% polyphenols by weight of dry matter.

8. A composition for use according to any of the preceding claims, **characterised in that** it is a dietary food for special medical purposes.

9. A composition for use according to any of the preceding claims, **characterised in that** it also comprises proteins, carbohydrates, lipids, vitamins and/or minerals.

10. A composition for use according to any of the preceding claims, **characterised in that** it is in powder form.

11. A composition for use according to any of the preceding claims, **characterised in that** it is administered in addition to and/or in substitution for meals.
